# EUROPEAN PATENT APPLICATION

(11) **EP 3 398 593 A1**
(43) Date of publication of application: **07.11.2018**
(21) Application number: 17305520.3
(22) Date of filing: 05.05.2017
(51) Int. Cl.: A61K 31/167, A61K 31/196, A61K 31/7008, A61K 33/18, A61K 45/06, A61P 39/00

(54) **IODINATED CONTRAST MEDIUM FOR USE AS A MEDICAMENT FOR THYROID RADIOPROTECTION**

(71) Applicant: COMMISSARIAT À L'ÉNERGIE ATOMIQUE ET AUX ÉNERGIES ALTERNATIVES, 75015 Paris (FR); Université Nice - Sophia Antipolis, 06103 Nice (FR)
(72) Inventor: POURCHER, Thierry, 06107 Nice (FR); CAMBIEN, Béatrice, 06107 Nice (FR); VASSAUX, Georges, 06107 Nice (FR)
(74) Representative: Santarelli

(57) **Abstract**

The present invention relates to a iodinated contrast medium (ICM), for use as a medicament for preventing radioactive iodine uptake by the thyroid gland after a nuclear accident. The ICM can advantageously be administered via the sublingual route.

## Description

### FIELD OF THE INVENTION

The present invention pertains to the field of radioprotection. More specifically, the invention pertains to a new use of iodinated compounds, for protecting the thyroid gland and its surrounding tissues from radioactive isotopes of iodine (I-131) released during a nuclear accident or administered to a human patient as part of a treatment targeting extra-thyroidal tissues.

### BACKGROUND AND PRIOR ART

The thyroid is an endocrine gland that synthesizes the iodine-containing thyroid hormones in vertebrates. Thyroid hormones are essential for the proper growth, multiplication and differentiation of all cells in the body, and to varying degrees, regulate the basal metabolism of proteins, lipids and carbohydrates. Iodine is an essential component in the synthesis of thyroid hormones. In the thyroid, iodide is taken up from the blood plasma by follicular cells. This uptake is mediated by the Na/I symporter (NIS) protein present at the basolateral membrane of these cells. Once inside the cells, free iodide is oxidized at the apical membrane, where it is covalently bound to tyrosine residues of the thyroglobulin protein. In addition to thyroid tissues, iodide uptake activity is observed in extra-thyroidal tissues such as salivary glands, gastric mucosa, lactating mammary gland, nasal mucosa, placenta and lacrimal glands. In these extra-thyroidal tissues, iodide is not organified.

In the event of a nuclear reactor accident and when radioactive material is released to the atmosphere, 131-I may be incorporated into the human body through inhalation or ingestion of contaminated food. About 10-30% of the inhaled radioactive iodine primarily accumulates in the thyroid gland, while the remaining amount is discharged from the body with the urine. Beta-radiation emitted upon decay of 131-I affects the thyroid and its surrounding tissues and leads to adverse health outcomes such as thyroid dysfunctions and thyroid cancer.

The well-established preventive countermeasure to protect populations in the event of an accidental release of radioactive iodine is ingestion of potassium iodide (KI) tablets. The administration of KI tablets prevents entry of 131-I into the thyroid. Mechanistically, this effect is the result of two phenomena. First, the high input of "cold" iodide dilutes 131-I, resulting in a competitive effect that reduces the uptake of 131-I. Second, this high iodide concentration triggers a physiological response, referred to as the Wolff-Chaikoff effect, in which the expression of the NIS protein is reduced. Nevertheless, the effect of KI tablets is transient and the protective effect only lasts for 24 hours. As a result, the United States Food and Drug Administration recommends KI tablets to be ingested on a daily basis in cases of prolonged 131-I exposure.

In cases of prolonged 131-I exposure of a large population, potential logistic and compliance problems may arise. In this context, "one shot" measures that could replace KI tablets or reduce the requirement for a stringent compliance to the daily intake of KI tablets would be welcome.

Another situation in which the radioprotection of the thyroid gland is necessary is that of administration of radioactive iodine in the frame of a treatment, for example an antineoplastic treatment targeting a tissue different from the thyroid gland. Iodine 131 therapy consists in the administration of iodine 131 to patients in order to eradicate differentiated thyroid carcinoma. One strategy is based on the ability of thyroid follicular cells to uptake and accumulate iodine (via the NIS symporter) to synthetize hormones. In the last fifteen years, iodine 131 therapies have been identified as a novel promising therapeutic strategy for the treatment of non-thyroidal tumors. Such a therapy comprises NIS gene transfer into the cancer cells, followed by therapeutic administration of radioiodine. The capacity of the NIS gene to induce radioiodine accumulation in non-thyroidal tumors has been investigated in a variety of tumor models such as prostate (Barton KN. *et al*., 2008) colorectal (Peerlinck I. *et al.,* 2009) and mammary gland cancer (Boland A. *et al*., 2000). Clinical studies in prostate cancer have shown the feasibility of this approach (Barton KN. *et al*., 2011). In this context, because of the endogenous NIS expression, radioiodine uptake by the thyroid gland was observed.

Another strategy for using radioiodine for targeting cancer cells is the use of radionuclides conjugated to tumor-directed monoclonal antibodies or peptides. Radioiodine conjugated to immunoglobulins or peptides can target tumor cells and locally deliver tumor-killing radiation. A recent study reports the use of radio-labeled (131) I-Metaiodobenzylguanidine (MIBG) to treat children with neuroblastoma (Clement S.C. *et al*., 2013). Thyroid gland uptake of radioiodine was blocked by oral administration of KI solution given before and after (131) I-MIBG infusion. Despite the protection with KI during exposure to (131) I-MIBG in childhood, radioiodine uptake in the thyroid gland was observed.

In this context, ensuring tumor specificity of radiation exposure is key, and a compound able to provide an efficient protection of the thyroid gland to radioiodine, with a longer protective window than KI, would be very useful.

Iodinated contrast media (ICM), also called iodinated contrast agents (ICA), are routinely administered to patients. ICM have been in use since the 1950s to facilitate radiographic imaging modalities (Jeffrey *et al*., 2012). All ICM share a similar function group - a tri-iodinated benzene ring. Iodine plays a key role in the attenuation of x-rays.

Two major chemical variations result in 4 classes of ICM. Compounds comprise either a unique tri-iodinated benzene ring (*i.e.*, monomers) or 2 tri-iodinated benzene rings linked by an organic functional group (*i.e.*, dimers). In addition, ionic tendency is governed by the presence (*i.e.,* ionic) or absence (*i.e.*, nonionic) of a carboxylate (-COO⁻) functional group contained on an organic side chain. Anionic ICM are usually available as salts of sodium, calcium, or methylglucamine cations. Hence, ICM can be classified in 4 classes:
1- Ionic monomer: single tri-iodinated benzene ring with a carboxylate-containing benzene substituent.
2- Ionic dimer: 2 linked tri-iodinated benzene rings in which at least 1 carboxylate-containing group is substituted on at least 1 benzene ring.
3- Nonionic monomer: single tri-iodinated benzene ring without a carboxylate-containing benzene substituent.
4- Nonionic dimer: 2 linked tri-iodinated benzene rings that do not contain a carboxylate functional group within any benzene substituent.

Considering their widespread utilization, ICM can be considered as safe. However, a well-documented side effect of intravenous ICM administration observed in most patients is compromise of diagnostic thyroid scintigraphy and radioiodine treatment of thyroid malignancies. For the latter, guidelines recommend delaying radioactive iodide treatment in patients who have been exposed to ICM. Mechanistically, this reduced iodide uptake by thyroid tissues in response to ICM is thought to be the result of injection of high amounts of free iodide contaminating the ICM formulation (Laurie *et al*., 1992). Many studies have reported that urinary iodide concentration remains high for days and even weeks after ICM administration (Padovani *et al.,* 2012; Lee *et al*., 2015). This high urinary iodide concentration may reflect a high blood iodide content that could be consistent with the long-lasting effect of ICM on thyroid iodide uptake. The source of this free iodide may be free iodide associated with ICM (Laurie *et al*., 1992) and/or released from ICM upon injection (van der Molen, 2004; Talner *et al.*, 1973).

ICM are predominantly administered by intravascular administration, although enteric administration and direct injection (e.g. in a cystography or in a sonography) can also be performed. ICM are administered in clinical locations only, and only for radiology purposes.

### SUMMARY OF THE INVENTION

The inventors have investigated the reasons of the reduced iodide uptake by the thyroid tissues following ICM administration. Unexpectedly, they demonstrated that this effect of ICM is not mediated by free iodide associated with and/or released from ICM, but that ICM induce thyroid stunning independently of free iodide, by triggering a specific and dramatic decrease in NIS expression in thyrocytes. Furthermore, they showed that this effect was also observed when ICM was administered via a sublingual route, which would be compatible with a wide administration to populations exposed to a radionuclear accident.

The present invention thus pertains to the use of a iodinated contrast medium (ICM) as a medicament for protecting the thyroid gland from radiation. According to the invention, this medicament can advantageously be administered through the sublingual or perlingual routes.

The invention also pertains to a therapeutic composition comprising an ICM and potassium iodide.

The invention also pertains to a kit of parts comprising, in separate formulations, potassium iodide and an ICM.

The invention also provides a therapeutic composition comprising a iodinated contrast medium and formulated for sublingual or perlingual administration.

### LEGENDS TO THE FIGURES

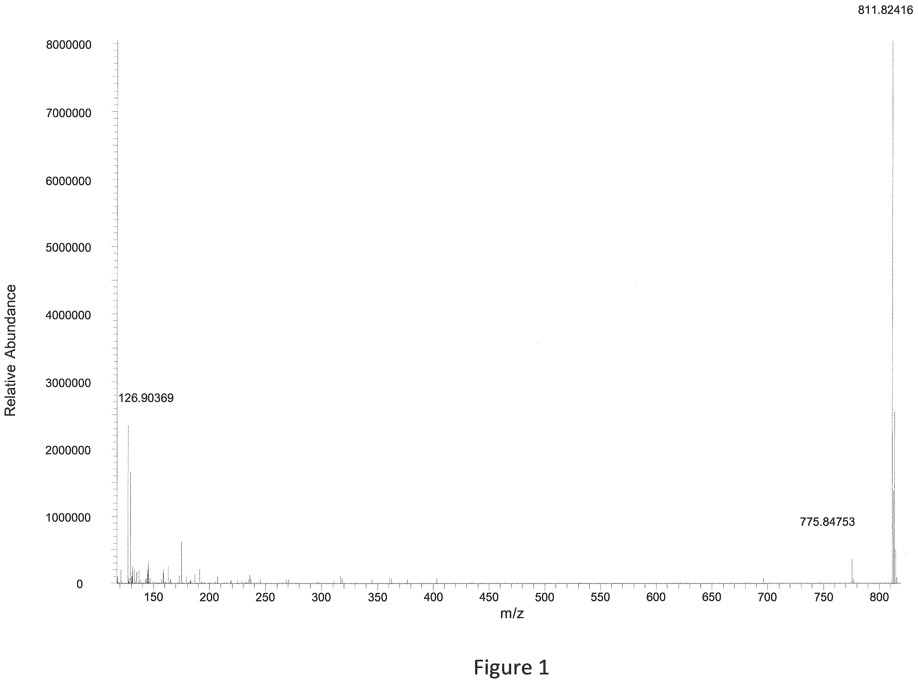

**Figure 1****: Mass spectrometry analysis of lomeron.** lomeron was diluted in 50/50 acetonitrile/H₂O to 1/10,000 (vol/vol) and characterized by high-resolution mass spectroscopy in negative mode electrospray. A major M-H+ peak at 775.84760 (experimental mass: 775.84573) corresponding to the formula C17H21O8N3I3 was observed.

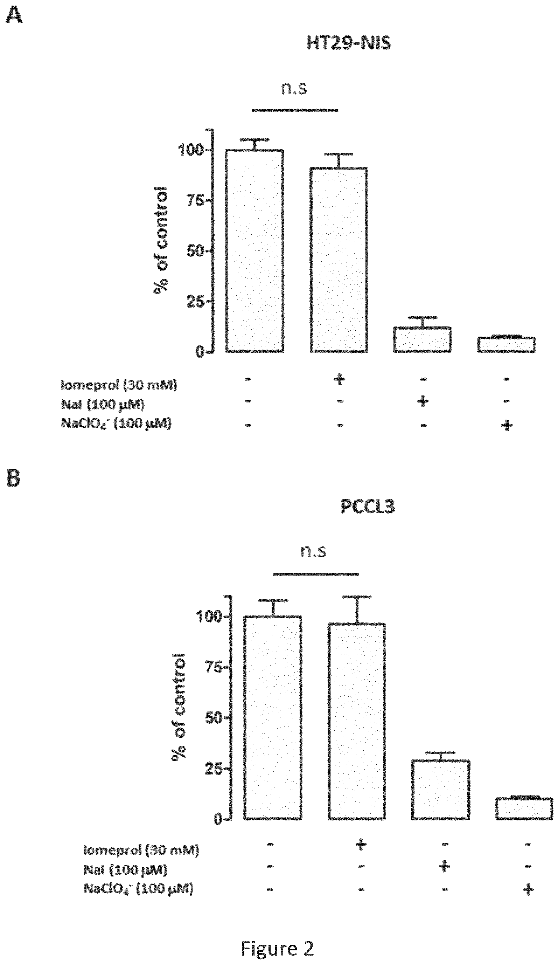

**Figure 2****: Effect of lomeron on iodide uptake of established cell lines expressing NIS.** HT29-NIS (A) or PCCL3 (B) cells were incubated for one hour with ¹²⁵I in the presence of an equal volume of either saline, lomeron (100 µL corresponding to 70 mg of iomeprol), Nal or perchlorate. Cells were then rapidly washed with saline buffer and lysed. Aliquots of lysates were counted in a y counter. The results are expressed as percentage of uptake in the control condition. The data presented are the mean ± SEM of triplicates and are representative of three independent experiments. n.s: non statistically significant.

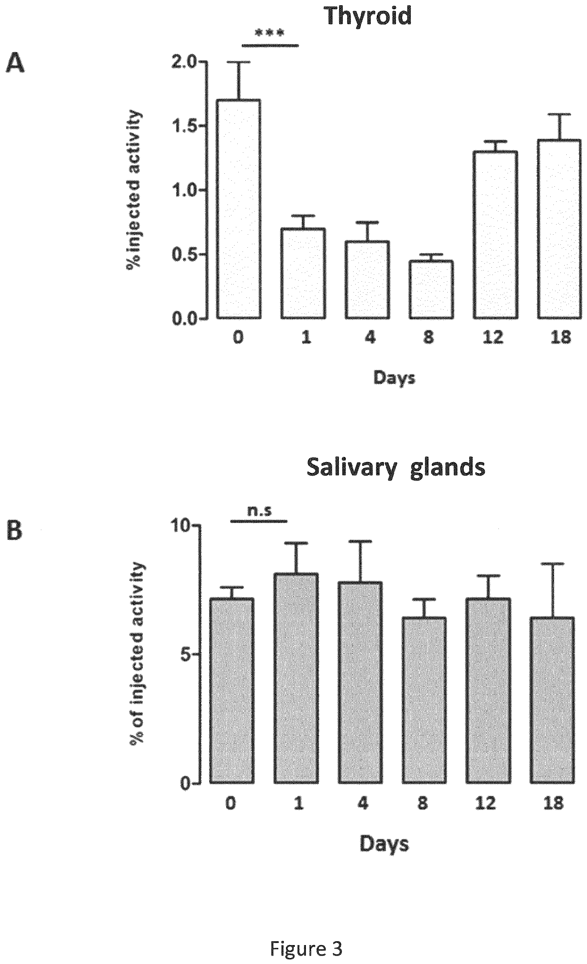

**Figure 3****: Effect of lomeron on the uptake of ^{99m}-perctechnetate by the mouse thyroid and salivary glands.** SPECT/CT imaging of mice administered 20 MBq **^{99m}-pertechnetate** was performed (day 0). At the end of the scan, lomeron was administered intravenously. One, four, eight, twelve and eighteen days later, animals were injected with 20 MBq ^{99m}-pertechnetate and new scans were performed. The data presented represent the percentage of radiotracer injected taken up by the thyroid (A) and salivary glands (B). (n = 3 per condition). *** p < 0.001; n.s: non statistically significant.

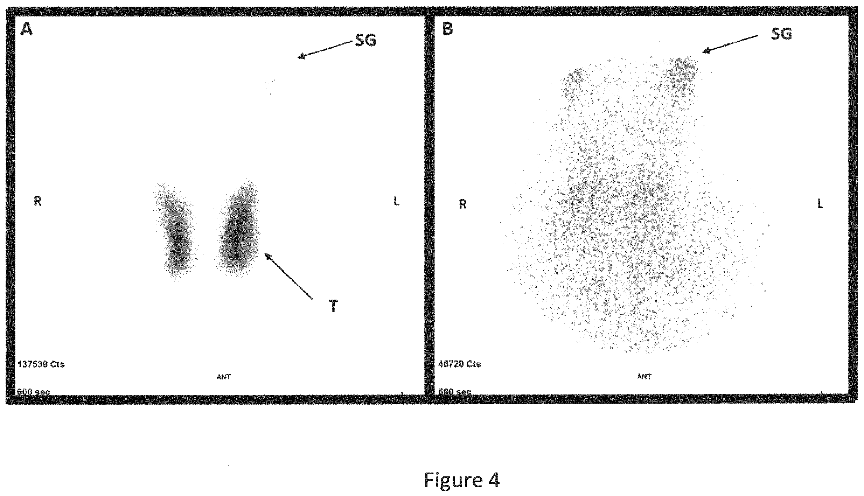

**Figure 4****: Effect of lomeron on the uptake of ^{99m}-perctechnetate by the human thyroid and salivary glands.** Scintigraphies of a naïve patient (A) or a patient who has been administered with lomeron two weeks before the scintigraphy. SG: salivary glands; T: thyroid; R: right; L: left.

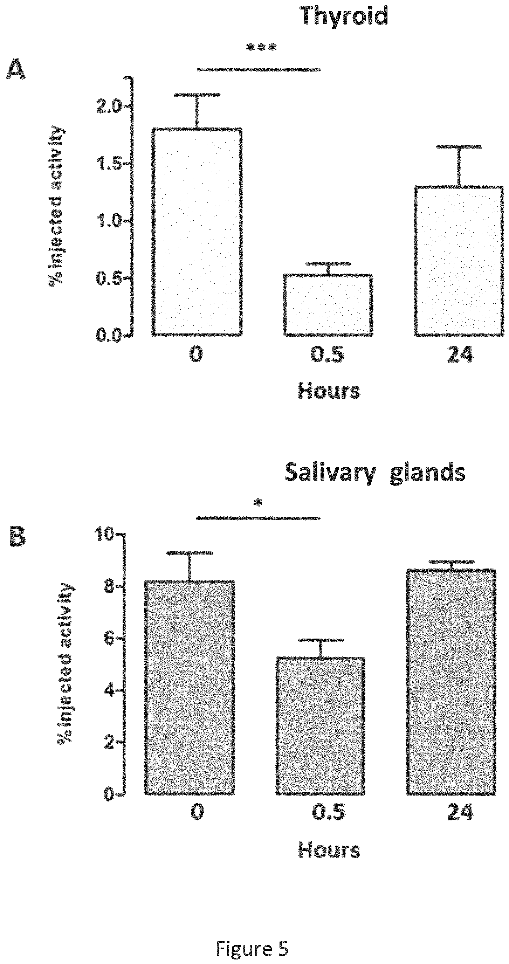

**Figure 5****: Effect of KI on the uptake of ^{99m}-perctechnetate by the mouse thyroid and salivary glands.** SPECT/CT imaging of mice administered 20 MBq ^{99m}-pertechnetate was performed (day 0). Animals were injected intraperitoneally with KI (9 mg in 180 µL) and scanned 30 minutes later. Twenty-four hours later, animals were injected with 20 MBq ^{99m}-pertechnetate and new scans were performed. The data presented represent the percentage of radiotracer injected taken up by the thyroid (A) and salivary glands (B). (n = 3 per condition). * p < 0.05; *** p < 0.001.

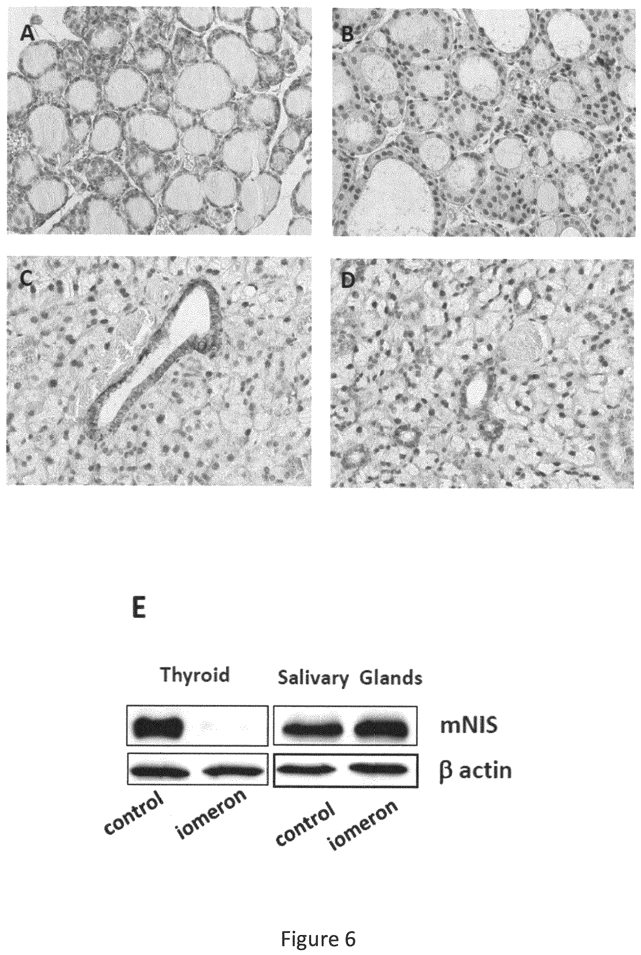

**Figure 6****: Analysis of NIS expression in the thyroid and salivary glands in response to lomeron.** Saline buffer (A, C) or lomeron (B, D) were administered intravenously. Four days later, animals were culled and NIS expression in the thyroid glands (A, B) and salivary glands (C, D) were analyzed by immunohistochemistry (n = 3 per group). Thyroids and salivary glands were also processed for Western blot analysis of NIS and β-actin expression (n = 2 per group).

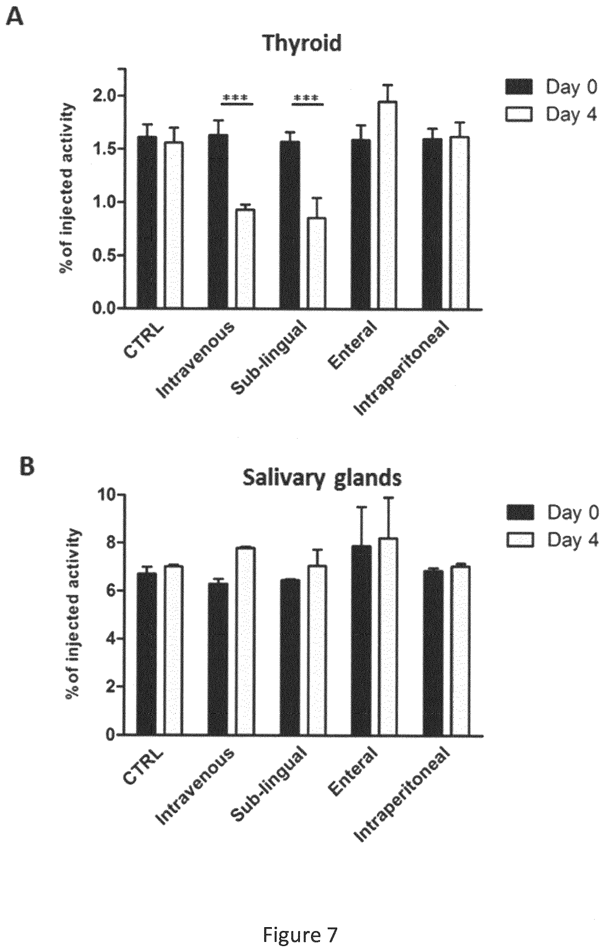

**Figure 7****: Evaluation of the effect of different modes of administration of lomeron on the uptake of ^{99m}-perctechnetate by the thyroid.** SPECT/CT imaging of mice administered 20 MBq ^{99m}-pertechnetate was performed (day 0). At the end of the scan, intravenous, sublingual, enteral, and intraperitoneal administrations of lomeron were performed. Four days later, animals were injected with 20 MBq ^{99m}-pertechnetate and new scans were performed. The data presented represent the percentage of radiotracer injected taken up by the thyroid (A) and salivary glands (B). (n = 2 per condition). *** p < 0.001.

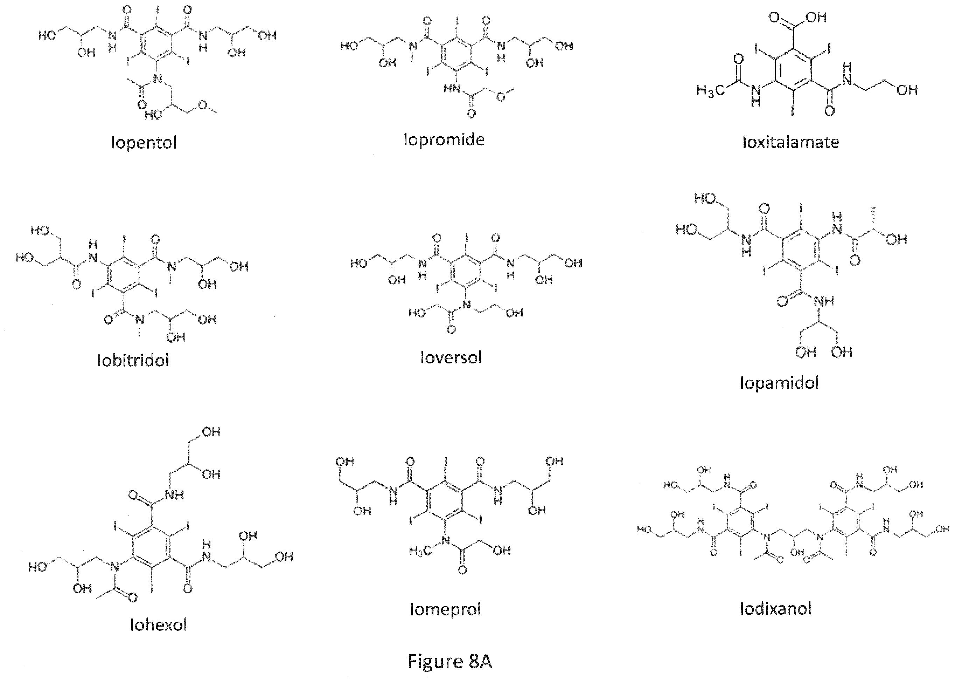

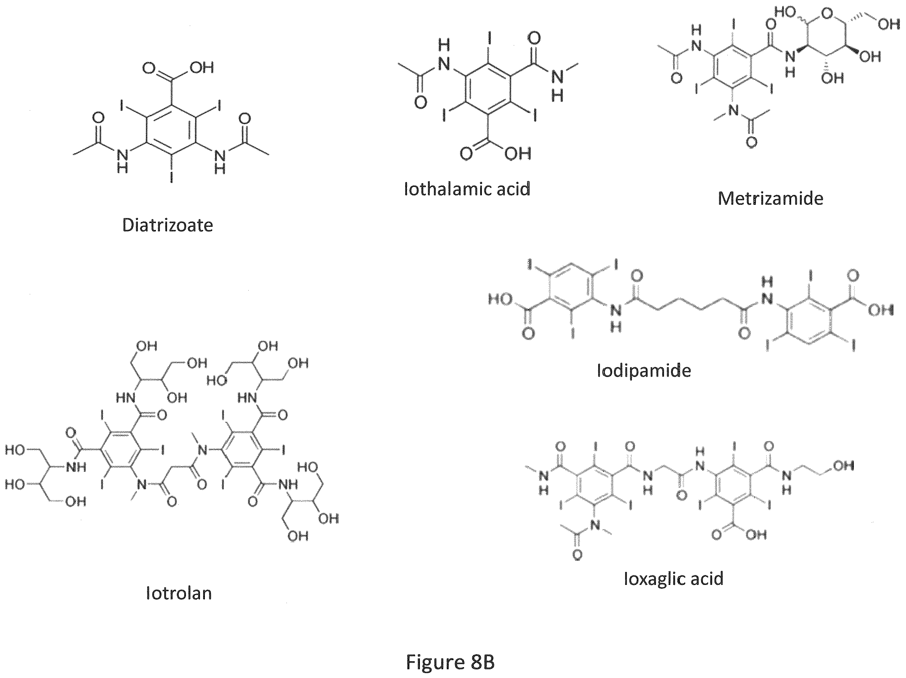

**Figure 8****: Formulas of ICMs that can be used for performing the present invention.**

### DETAILED DESCRIPTION OF THE PREFERRED EMBODIMENTS

According to a first embodiment, the present invention pertains to the use of iodinated contrast medium (ICM), as a medicament for protecting the thyroid gland from radiation. This protection is achieved by preventing thyroid uptake of radioiodine. A treatment for preventing thyroid disorders caused by radioiodine, comprising administering an ICM to a subject exposed to radioactive iodine, is also part of the invention.

In the present text, a "iodinated contrast medium" designates any compound comprising a tri-iodinated benzene ring and that can be used as a contrast agent in radiology.

According to a preferred embodiment of the invention, the ICM comprises a triiodophenyl derivative and several hydrophilic groups. Non-limitative examples of ICM that can be used in the frame of the present invention are iodixanol, iomeprol, iohexol, iopamidol, ioversol, iobitridol, iopromide, iopentol, ioxitalamate, iodipamide, metrizamide, iotrolan, iothalamate, diatrizoate and ioxaglate (Fig. 8).

As mentioned above, ICMs can be classified in 4 different categories, depending on the number of tri-iodinated benzene ring(s) they comprise (one or two) and depending on their ionic or non-ionic nature. The experimental results described below have been obtained with iomeprol (non-ionic monomer) and similar results have been obtained by the inventors with iodixanol (non-ionic dimer - data not shown).

According to a particular embodiment of the invention, the ICM is a non-ionic organoiodine compound such as, for example, iomeprol or iodixanol.

According to yet another embodiment of the invention, the ICM has the formula I: 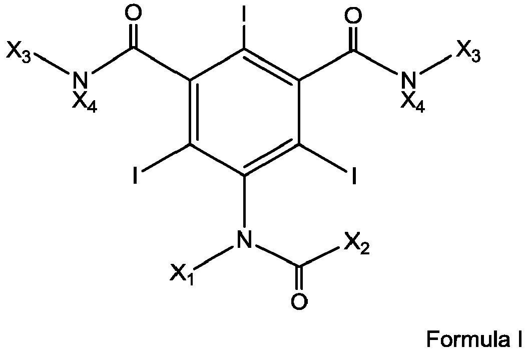 wherein:
- X₁ is selected from the group consisting of H, CH₃, CH₂OH, CH₂-CH₂OH, CH₂-CH(OH)-CH₂OH and CH₂-CH(OH)-CH₂-O-CH₃ or X₁ is an organic functional group linking two molecules of Formula I, such as, for example, a linker of formula -CH₂-CH(OH)-CH₂-;
- X₂ is selected from the group consisting of CH₃, CH₂OH, CH₂-O-CH₃, CH₂(OH)-CH₃ and 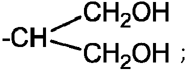
- X₂ is selected from the group consisting of CH₂-CH(OH)-CH₂OH and 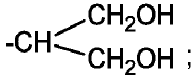 and
   X₄ is H or CH₃.

The inventors demonstrated that sublingual delivery of ICM is efficient in mice (Fig. 7). Contrary to humans who have a non-keratinized epithelium on the inferior surface of the tongue, mice have a keratinized mucosae on the inferior surface of the tongue, which may result in a physicochemical barrier against sub-lingually administered substances (Thirion-Delalande *et al.*, 2015). Sulingual delivery of ICM in humans should thus prove at least as efficient as that observed in mice, which opens the way to an easy administration to populations exposed to radioactive iodine, for example in the event of a nuclear accident.

According to another embodiment, the present invention thus pertains to the use of an ICM, as a medicament for protecting the thyroid gland from radiation, wherein said medicament is administered through the sublingual or perlingual route. A treatment for preventing thyroid disorders caused by radioiodine, comprising administering an ICM, through the sublingual or perlingual route, to a subject exposed to radioactive iodine, is also part of the invention.

According to a particular embodiment of the above-described invention, the ICM selectively inhibits iodine uptake by the thyroid gland. In particular, iodine uptake by the salivary glands is not affected by the ICM.

According to another particular embodiment of the above-described invention, the ICM selectively inhibits sodium/iodide symporter (NIS) expression in the thyroid gland.

The inventors observed that the effects of ICM on iodine uptake last far longer than those of KI. Indeed, as described in the experimental part below, the uptake of the iodide analog pertechnetate was dramatically reduced after ICM uptake, both in mice and in humans. Fig. 3A shows that this reduction was observed 24 hours after ICM administration and that uptake capacity remained low for at least eight days. This property is particularly interesting for obtaining a stable and long protective effect in situations where a radioiodine pollution lasts several days such as in the context of long-lasting nuclear accidents. The use of an ICM as a medicament for preventing radioactive iodine uptake by the thyroid gland after a nuclear accident is hence a particular embodiment of the present invention.

As mentioned above, radioiodine can be administered as an anticancer agent against extra-thyroidal tumors, either coupled to a targeting molecule such as an antibody or a ligand for a specific cell receptor expressed by tumor cells, or as free radioiodine in a radioiodine therapy following sodium iodide symporter gene transfer in cancer cells. In the latter case, the specificity can be achieved by the use of a gene transfer vector specifically targeting the tumor and/or by the use of a tissue-specific promoter for the expression of the Na/I symporter gene (for example, the carcinoembryonic antigen (CEA) promoter to target a colon cancer). In both cases however, uptake of radioactive iodide by the thyroid is to be avoided. According to yet another aspect, the present invention hence pertains to the use of an ICM as a medicament for protecting the thyroid gland from radiation in a cancer patient in need thereof, for example after administration of radioactive iodine as an antineoplastic agent.

When a prolonged protection is needed (more than 4 to 10 days - for example in the event of a nuclear event with prolonged radioiodine pollution or in the case of a long-lasting radioiodine therapy), the ICM can be administered once every 4 to 10 days, for example every 4, 5, 6, 7, 8, 9 or 10 days. Such a repeated administration is also part of the present invention.

According to another embodiment of the invention, the ICM is administered in combination with potassium iodide (KI). Combined administration of ICM and KI indeed enables both a rapid protective effect, due to KI, and a longer and stable protection due to the ICM. In the present text, "in combination" means that a subject receives both KI and an ICM, either simultaneously or sequentially. When the two compounds are administered separately, KI is preferably administered first.

The present invention also pertains to a therapeutic composition comprising a iodinated contrast medium as described above and potassium iodide. Such a composition according to the invention can be formulated in any convenient form, such as a solution, a pill, a tablet, pellets or globules *etc.* Preferred formulations are those which are compatible with sublingual or perlingual administration.

A kit of parts comprising, in separate formulations, potassium iodide and a iodinated contrast medium as described above, is also part of the present invention. In such a kit, KI can be formulated in the form of tablets and the ICM can be formulated in any convenient form, such as a solution, a pill, a tablet, pellets or globules *etc.* Preferred formulations are those which are compatible with sublingual or perlingual administration. A kit of parts according to the invention can also comprise a first tablet or pill or vial with pellets or globules or a solution comprising both KI and an ICM, and at least another tablet / pill / vial with pellets or globules or solution comprising only the ICM.

According to another aspect, the present invention relates to a therapeutic composition comprising a iodinated contrast medium as described above and a pharmaceutical vehicle appropriate and/or optimized for sublingual or perlingual administration. In particular, the therapeutic composition formulated for sublingual or perlingual administration can be in the form of a tablet, a syrup, a solution, pellets or globules. It can also comprise, in addition to the ICM, a flavor agent in order to render the uptake for pleasant.

Other characteristics of the invention will also become apparent in the course of the description which follows of the biological assays which have been performed in the framework of the invention and which provide it with the required experimental support, without limiting its scope.

### EXAMPLES

### Material and Methods

### Animals

Eight-week-old female C57B/6J mice were obtained from Janvier (Le Genest Saint Isle, France). Animal housing and procedures were conducted according to French Agriculture Ministry guidelines and were approved by the local ethics committee (Ciepal NCE/2014-211).

### Contrast agents

Experiments were performed using iomeprol (lomeron 350; lot LP4557) and iodixanol (Visipaque 320; lot 125 78 776).

### Cell lines

The human colorectal cancer cell line HT-29 (HTB-38, ATCC) was transfected with pcDNA3.1-mNIS (murine NIS) (Perron *et al.*, 2001) using the FuGENE 6 reagent (Roche) according to the manufacturer's instructions. Stable clones were selected by addition of 1 mg/mL geneticin (G418) to the medium 3 days after transfection. One clone with high functional expression of NIS was selected (Richard-Fiardo *et al.,* 2012). The rat follicular thyroid cell line PCCL3 was obtained from Dr. A. De La Vieja (Madrid, Spain) and cultured as previously described (Leoni *et al*., 2011). Measurement of *in vitro* iodide uptake was performed as previously described (Groot-Wassink *et al.*, 2002).

### Mass spectrometry

lomeron and Visipaque were diluted to 1/10,000 in 50/50 (vol/vol) acetonitrile/H₂O and characterized by high resolution mass spectroscopy in negative mode electrospray. For the direct-infusion experiments, a flow of 5 µL/min was provided by a syringe pump (11 Plus, Harvard Apparatus, Holliston, MA, USA) using a 500 µL/min syringe (Hamilton, Reno, NV, USA). The mass spectrometer (Q Exactive Plus, Thermo Fisher Scientific, Bremen, Germany) was operated with an electrospray source for the direct-infusion method development. The direct-infusion Orbitrap measurements were carried out using the Ion Max source from Thermo Fisher Scientific and applying the following parameters: sheath gas flow, 15 arbitrary units; auxiliary gas flow, 5 arbitrary units; capillary temperature, 275°C. The automatic gain control target was set to 10⁶ and the maximum injection time was 50 ms. In the high-resolution measurements with setting of 140 000 at m/z 200 one microscan was recorded. The spray voltage in negative mode was selected at -2.5 kV.

### Administration of ICM in mice in vivo

Intravenous and intraperitoneal administration was performed by injecting 100 µL lomeron diluted 50/50 (vol/vol) with phosphate-buffered saline (corresponding to 18 mg iomeprol). Enteral administration was performed by gavage of 100 µL lomeron diluted 50/50 (vol/vol) with phosphate-buffered saline (corresponding to 18 mg iomeprol). Sublingual administration was performed on anesthetized mice by five sublingual depositions of 5 µL lomeron, with a delay of 10 minutes between two administrations. This procedure led to the administration of 9 mg iomeprol.

### In vivo microSPECT/CT studies

^{99m}Tc pertechnetate (^{99m}TcO₄⁻) was obtained from a freshly eluted ⁹⁹Mo/^{99m}Tc generator. Animals were administered intraperitoneally with activities of 20 MBq ^{99m}TcO₄⁻. Thyroid and salivary gland tracer uptake was measured at different times using a dedicated microSPECT/CT scanner (eXplore speCZT CT120, GE) under gas anesthesia (air and 1-2% isoflurane) in an air-warmed imaging chamber (Minerve, Esternay, France) to maintain body temperature at 37°C. The SPECT scanner uses a stationary full ring of CZT detectors and a rotating 7-pinhole (1 mm opening) collimator. A total of 350 projections were acquired over 360° in 8 minutes. Images were reconstructed using the manufacturer's 3D-OSEM algorithm (5 subsets and 11 iterations), which incorporates the system's collimator-detector response function and scatter correction. Reconstructed images were analyzed and quantified using AMIDE software (Loening and Gambhir, 2003). Tracer activities in the various relevant organs were calculated. 3D regions of interest (ROIs) were drawn manually around the thyroid and salivary glands, as previously detailed (Richard-Fiardo *et al*., 2015; Zwarthoed *et al*., 2016). Uptakes were expressed as percentages of the injected dose (%ID) after decay correction (Cambien *et al.*, 2014).

### Thyroid and salivary uptake of ^{99m}TcO₄⁻ in humans

A "naïve" patient and a patient who had received an intravenous injection of lomeron 350 two weeks before scintigraphy were involved. On the day of the scintigraphy, patients were injected intravenously with an activity of 1 MBq/kg and 600 s scans was performed 15 min later.

### Membrane vesicle preparation, SDS-PAGE and Western blot analyses

Thyroid membrane proteins were subjected to SDS-PAGE electrophoresis as previously described (Cambien *et al*., 2014). Western blotting was performed with antibody 25 anti-mouse NIS, an affinity-purified rabbit immunoreactive serum fraction, or with an anti-β-actin antibody (Sigma).

### Immunohistochemistry

After culling the animals, thyroids and salivary glands were dissected, paraffin embedded, and cut into 4-µm-thick sections. The paraffin was then removed and the sections were rehydrated and subjected to an antigen retrieval treatment with a solution of citrate buffer, pH 6, using an automate (PT Link, Dako). Immunostaining was performed following a standard protocol (Dako EnVisionTM FLEX using an automated immunostainer, Autostainer, Dako). Endogenous peroxide was blocked using the EnVisionTM FLEX Peroxidase-Blocking Reagent. After pretreatment, slides were incubated for 20 minutes at room temperature with a rabbit polyclonal antibody against NIS (antibody 25, see Dayem *et al*., 2008) at a 1:200 dilution. NIS immunostaining was performed with a secondary anti-mouse antibody and rabbit/HRP (Dako, DM822) using a 3,3' -diaminobenzidine (DAB) cosubstrate (Dayem *et al*., 2008). The DAB-stained sections were counterstained with Harris hematoxylin (Sigma, Saint Quentin Fallavier, France). Image acquisition was performed using a Nikon 80i microscope equipped with a DS-5M-L1 digital camera.

### Statistical analysis

Statistical analysis was performed using Prism (GraphPad software). Dual comparisons were made using a Student's t-test and comparisons between multiple conditions were analyzed using ANOVA. Statistical significance was set at P < 0.05.

### Results

### Free iodide content of ICM

We first evaluated whether free iodide was present in commercially available ICM. Mass spectrometry analysis of a commercially-available solution of lomeron (Fig. 1) revealed the presence of a peak at M-H- at 126.90369 corresponding to free iodide in addition to a peak at 775.84573 corresponding to iomeprol. These data demonstrate the presence of free iodide in the lomeron formulation. Spectrometric analysis of Visipaque (not shown) led to a similar conclusion.

### Effect of ICM on the uptake of iodide in cell lines

We next examined whether iomeprol and its contaminating free iodide could affect iodide uptake in NIS-expressing cell lines. HT29-NIS (a human colorectal carcinoma cell line expressing NIS) (Fig. 2A) or PCCL3 (a rat follicular thyroid cell line) (Fig. 2B) cells were incubated with ¹²⁵I in the presence of either saline buffer, lomeron, Nal, or perchlorate (NaClO₄). After one hour, cells were washed and cellular ¹²⁵I content was determined. As expected, both Nal and perchlorate inhibited iodide uptake in both cell lines. By contrast, lomeron failed to affect this uptake significantly.

### Effect of ICM on pertechnetate uptake in vivo

We evaluated whether lomeron could affect the uptake of the iodide analog, pertechnetate, by the mouse thyroid and the salivary glands. Basal activities were measured before (TO) and after intravenous administration of lomeron. Figure 3A shows that a dramatic reduction in radiotracer uptake by the thyroid was observed 24 hours after lomeron administration. This uptake capacity remained low for eight days and a recovery was observed from day 12. By contrast, in the same animals, lomeron administration failed to affect iodide uptake by another NIS-expressing tissue, the salivary glands (Fig. 3B). Similar data were obtained using Visipaque (not shown). This observation suggests that ICM affect the mouse thyroid and the salivary glands differently. To evaluate whether this differential action is observed in humans, we compared pertechnetate uptake in the thyroid and salivary glands of a naïve patient (Fig. 4A) or a patient who had received an intravenous injection of lomeron two weeks before (Fig. 4B). Figure 4A shows the scintigraphy of a naïve patient in which both the thyroid and salivary glands are taking up pertechnetate. By contrast, the scintigraphy of a patient treated with lomeron shows radiotracer uptake in the salivary glands and a lack of fixation in the thyroid region (Fig. 4B).

### Effect of potassium iodide on pertechnetate uptake in vivo

A similar experiment was carried out to evaluate the effect of KI on radiotracer uptake by the thyroid and salivary glands. Figure 5A shows that the ability of the thyroid to take up pertechnetate was reduced 30 minutes after intraperitoneal injection of KI. This capacity was recovered 24 hours after injection. A similar pattern was observed when pertechnetate uptake to the salivary glands was measured (Fig. 5B). This dataset demonstrates that potassium iodide affects thyroidal and salivary gland pertechnetate uptake equally.

### Effect of ICM on NIS expression in vivo

We next evaluated the effect of lomeron on NIS expression in the thyroid and salivary glands. Immunohistological analysis revealed that NIS expression was hardly detectable in thyroids four days after lomeron injection (Fig 6B), as compared with control mice (Fig. 6A). By contrast, NIS expression was detectable in the ductal cells of the salivary glands of both control and lomeron-treated mice (Fig. 6C and D). Semi-quantitative analysis by western blot confirmed a dramatic decrease in NIS expression in the thyroid glands of lomeron-treated mice compared with controls (Fig.6E). By contrast, no significant difference between NIS expression was detected in the salivary glands in both groups.

### Mode of administration of ICM

We next compared the efficacy of different modes of administration of lomeron on pertechnetate uptake by the thyroid and the salivary glands. Figure 7A shows that neither enteral nor intraperitoneal administration affected radiotracer uptake by the thyroid. By contrast, intravenous and sublingual administration of the ICM resulted in a marked reduction in radiotracer uptake (Fig. 7A). Uptake to the salivary glands was not affected in any condition (Fig. 7B).

### Discussion

One side effect of the utilization of ICM is alteration of iodide uptake by the thyroid. This perturbation has been attributed to the free iodide associated with and/or released from ICM on administration. We first determined whether free iodide contamination associated with the ICM could mediate this effect. Mass spectrometric analysis demonstrated the presence of significant amounts of free iodide in the lomeron (iomeprol) (Fig. 1) and Visipaque (iodixanol) (not shown) formulations. However, competition experiments using NIS-expressing cells demonstrated that lomeron did not affect iodide uptake (Fig. 2). Overall, these *in vitro* results suggest that if free iodide is involved in the perturbation of iodide uptake by the thyroid, it must be released in the body from the deiodination of ICM.

Free iodide perturbed uptake of the iodide analog, ^{99m}-pertechnetate, by the mouse thyroid and salivary glands to an equivalent extent (Fig. 5). Kinetically, for both organs, uptake decreased rapidly (30 minutes after KI injection in our experiment), and the capacity of the thyroid to take up the radiotracer was restored within 24 hours. By contrast, iomeprol affected uptake of ^{99m}-pertechnetate to the thyroid selectively (Fig. 3). This differential effect of the ICM on the thyroid and salivary glands was also observed in patients (Fig.4). Overall, this dataset rules out a direct intervention of free iodide in the perturbation of uptake to the thyroid and suggests a direct and selective effect of iomeprol on the thyroid. Considering that similar data were obtained with iodixanol (not shown), ICM, in general, may act directly and selectively on the thyroid.

The mechanism by which ICM reduces iodide uptake by the thyroid involves a dramatic reduction in the expression of the NIS protein. Four days after ICM injection, the NIS protein was hardly detectable using immunohistochemistry. Western blot analysis confirmed this observation. As expected from the functional imaging data, NIS protein expression was unaffected in the salivary glands. To exert this effect, it is likely that ICM trigger an intracellular molecular mechanism that results in reduced NIS protein expression.

Although effective and safe overall, the utilization of KI tablets to protect populations in the event of a nuclear incident is associated with some problematic issues, as KI tablets need to be ingested on a daily basis. In cases of prolonged 131-I exposure of a large population, potential logistic and compliance problems may arise. In this context, "one shot" measures that could replace KI tablets or reduce the requirement for a stringent compliance to the daily intake of KI tablets would be welcome. Formulations based on ICM could provide this benefit. Given that the intravenous mode of administration is hardly relevant for an application in radioprotection, the sublingual delivery of ICM is efficient in mice and should also be efficient in humans. In addition, ICM could be chemically modified to be absorbed by the gut epithelium, leading the way to a compound administered orally.

### REFERENCES

Barton KN, Stricker H, Brown SL, Elshaikh M, Aref I, Lu M, et al. Phase I study of noninvasive imaging of adenovirus-mediated gene expression in the human prostate. Mol Ther. 2008;16(10):1761 9.
Barton KN, Stricker H, Elshaikh MA, Pegg J, Cheng J, Zhang Y, et al. Feasibility of adenovirus-mediated hNIS gene transfer and 1311 radioiodine therapy as a definitive treatment for localized prostate cancer. Mol Ther. 2011;19(7):1353 9.
Boland A., Rchard M., Opolon P., Bidart J.-M., Yeh P., Filetti S., Schlumberger M. and Michel Perricaudet. Adenovirus-mediated Transfert of the Thyroid Sodium/Iodide Symporter Gene into Tumors for a Targeted Radiotherapy. Cancer Res. July 2000; 60, 3484-3492.
Cambien B, Franken PR, Lamit A, et al. (9)(9)mTcO(4)--, auger-mediated thyroid stunning: dosimetric requirements and associated molecular events. PLoS One. 2014;9(3):e92729.
Clement S.C., B.L.F. van Eck-Smit, A.S.P. van Trotsenburg, L.C.M. Kremer, G.A.M. Tytgat, and H.M. van Santen, Long-Term Follow-Up of the Thyroid Gland After Treatment With 131I-Metaiodobenzylguanidine in Children With Neuroblastoma: Importance of Continuous Surveillance. Pediatr Blood Cancer 2013;60:1833-1838.
Dayem M, Basquin C, Navarro V, et al. Comparison of expressed human and mouse sodium/iodide symporters reveals differences in transport properties and subcellular localization. J Endocrinol. Apr 2008;197(1):95-109.
Groot-Wassink T, Aboagye EO, Glaser M, Lemoine NR, Vassaux G. Adenovirus biodistribution and noninvasive imaging of gene expression in vivo by positron emission tomography using human sodium/iodide symporter as reporter gene. Hum Gene Ther. Sep 20 2002;13(14):1723-1735.
Hingorani M, Spitzweg C, Vassaux G, et al. The biology of the sodium iodide symporter and its potential for targeted gene delivery. Curr Cancer Drug Targets. Mar 2010;10(2):242-267.
Jeffrey J. Pasternaka and Eric E. Williamson. Clinical Pharmacology, Uses, and Adverse Reactions of lodinated Contrast Agents: A Primer for the Non-radiologist. Mayo Clin Proc. 2012 Apr; 87(4): 390-402.
Laurie AJ, Lyon SG, Lasser EC. Contrast material iodides: potential effects on radioactive iodine thyroid uptake. J Nucl Med. Feb 1992;33(2):237-238.
Lee SY, Chang DL, He X, Pearce EN, Braverman LE, Leung AM. Urinary iodine excretion and serum thyroid function in adults after iodinated contrast administration. Thyroid. May 2015;25(5):471-477.
Leoni SG, Kimura ET, Santisteban P, De la Vieja A. Regulation of thyroid oxidative state by thioredoxin reductase has a crucial role in thyroid responses to iodide excess. Mol Endocrinol. Nov 2011;25(11):1924-1935.
Loening AM, Gambhir SS. AMIDE: a free software tool for multimodality medical image analysis. Mol Imaging. Jul 2003;2(3):131-137.
Padovani RP, Kasamatsu TS, Nakabashi CC, et al. One month is sufficient for urinary iodine to return to its baseline value after the use of water-soluble iodinated contrast agents in post-thyroidectomy patients requiring radioiodine therapy. Thyroid. Sep 2012;22(9):926-930.
Peerlinck I, Merron A, Baril P, Conchon S, Martin-Duque P, Hindorf C, et al. Targeted radionuclide therapy using a Wnt-targeted replicating adenovirus encoding the Na/I symporter. Clin Cancer Res. 2009;15(21):6595 601.
Perron B, Rodriguez AM, Leblanc G, Pourcher T. Cloning of the mouse sodium iodide symporter and its expression in the mammary gland and other tissues. J Endocrinol. Jul 2001;170(1):185-196.
Richard-Fiardo P, Franken PR, Lamit A, et al. Normalisation to blood activity is required for the accurate quantification of Na/I symporter ectopic expression by SPECT/CT in individual subjects. PLoS One. 2012;7(3):e34086.
Richard-Fiardo P, Hervouet C, Marsault R, et al. Evaluation of tetrafunctional block copolymers as synthetic vectors for lung gene transfer. Biomaterials. Mar 2015;45:10-17.
Talner LB, Coel MN, Lang JH. Salivary secretion of iodine after urography. Further evidence for in vivo deiodination and salivary secretion of contrast media. Radiology. Feb 1973;106(2):263-268.
Thirion-Delalande Catherine, Fish C., Forster R., Palate, B. Comparative histology of mouth mucosae (sublingual region). Toxicology Letters. October 2015; 238(2):S271-S272.
Van der Molen AJ, Thomsen HS, Morcos SK. Effect of iodinated contrast media on thyroid function in adults. Eur Radiol. May 2004;14(5):902-907.
Zwarthoed C, Chatti K, Guglielmi J, et al. Single-Photon Emission Computed Tomography for Preclinical Assessment of Thyroid Radioiodide Uptake Following Various Combinations of Preparative Measures. Thyroid. Nov 2016;26(11):1614-1622.

## Claims

1. A iodinated contrast medium (ICM), for use as a medicament for protecting the thyroid gland from radiation.

2. The ICM of claim 1, for the use of claim 1, wherein said medicament is administered through the sublingual or perlingual route.

3. The ICM of claim 1 or claim 2, for the use of claim 1 or claim 2, wherein said ICM comprises a triiodophenyl derivative and several hydrophilic groups.

4. The ICM of any of claims 1 to 3, for the use of claim 1 or claim 2, wherein said ICM is selected from the group consisting of iodixanol, iomeprol, iohexol, iopamidol, ioversol, iobitridol, iopromide, iopentol, ioxitalamate, iodipamide, metrizamide, iotrolan, iothalamate, diatrizoate and ioxaglate.

5. The ICM of any of claims 1 to 4, for the use of claim 1 or claim 2, wherein said ICM is a non-ionic organoiodine compound.

6. The ICM of any of claims 1 to 5, for the use of claim 1 or claim 2, wherein said ICM has the formula I: 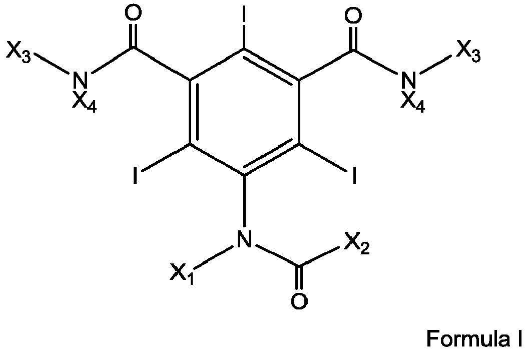 wherein:
- X₁ is selected from the group consisting of H, CH₃, CH₂OH, CH₂-CH₂OH, CH₂-CH(OH)-CH₂OH and CH₂-CH(OH)-CH₂-O-CH₃ or X₁ is an organic functional group linking two molecules of Formula I;
- X₂ is selected from the group consisting of CH₃, CH₂OH, CH₂-O-CH₃, CH₂(OH)-CH₃ and 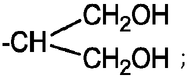
- X₂ is selected from the group consisting of CH₂-CH(OH)-CH₂OH and 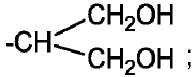 and
- X₄ is H or CH₃.

7. The ICM of any of claims 1 to 6, for the use of claim 1 or claim 2, wherein said ICM selectively inhibits iodine uptake by the thyroid gland.

8. The ICM of any of claims 1 to 6, for the use of any of claims 1, 2 and 7, wherein said ICM selectively inhibits sodium/iodide symporter (NIS) expression in the thyroid gland.

9. The ICM of any of claims 1 to 6, for the use of any of claims 1, 2, 7 and 8, wherein said ICM is administered in combination with potassium iodide (KI).

10. The ICM of any of claims 1 to 6, for the use of any of claims 1, 2 and 7 to 9, for use as a medicament for preventing radioactive iodine uptake by the thyroid gland after a nuclear accident.

11. The ICM of any of claims 1 to 6, for the use of any of claims 1, 2 and 7 to 9, for use as a medicament for preventing radioactive iodine uptake by the thyroid gland after administration of radioactive iodine as an antineoplastic agent.

12. The ICM of any of claims 1 to 6, for the use of any of claims 1, 2 and 7 to 11, wherein said ICM is administered once every 4 to 10 days.

13. A therapeutic composition comprising a iodinated contrast medium as recited in any of claims 1 to 6 and potassium iodide.

14. A kit of parts comprising, in separate formulations, potassium iodide and a iodinated contrast medium as recited in any of claims 1 to 6.

15. A therapeutic composition comprising a iodinated contrast medium as recited in any of claims 1 to 6 and a pharmaceutical vehicle for sublingual or perlingual administration.
